# EUROPEAN PATENT SPECIFICATION

(11) **EP 3 689 352 B1**
(45) Date of publication and mention of the grant of the patent: **15.11.2023**
(21) Application number: 20154086.1
(22) Date of filing: 09.02.2018
(51) Int. Cl.: A61K 31/4741, A61K 31/728, A61P 31/12

(54) **EVALUATION OF THE SEVERITY OF PATIENTS WITH FLAVIVIRUS INFECTION BY BLOOD HYALURONAN LEVELS AND THERAPEUTIC AGENTS TO BLOCK THE HYALURONAN**
BEWERTUNG DES SCHWEREGRADES VON PATIENTEN MIT FLAVIVIRUS-INFEKTION DURCH HYALURONANSPIEGEL IM BLUT UND THERAPEUTISCHE MITTEL ZUR BLOCKIERUNG DER HYALURONAN
ÉVALUATION DE LA GRAVITÉ DES PATIENTS ATTEINTS D'UNE INFECTION À FLAVIVIRUS PAR LES NIVEAUX D'HYALURONANE SANGUIN ET AGENTS THÉRAPEUTIQUES POUR BLOQUER L'HYALURONANE

(30) Priority: 05.10.2017 TW 106134411
(43) Date of publication of application: 05.08.2020
(62) Divisional of application: 18155997.2
(73) Proprietor: Kaohsiung Medical University, 807 Kaohsiung City (TW)
(72) Inventor: CHEN, Yen-Hsu, 807 Kaohsiung City (TW); LIN, Chun-Yu, 80767 Kaohsiung City (TW); HELDIN, Paraskevi, 751 24 Uppsala (SE)
(74) Representative: Isarpatent

(56) References cited:
- HONSAWEK ET AL: "Increased levels of serum hyaluronan in patients with dengue infection.", J. OF INFECTION., vol. 54, 28 July 1995 (1995-07-28), - 28 July 2005 (2005-07-28), pages 225-229, XP002783264,

## Description

The present invention is related to a pharmaceutical composition. In particular, the present invention is related to a pharmaceutical composition for blocking the serum hyaluronan (HA) in Dengue fever patients.

The viruses in the Flaviviridae family are single-stranded linear RNA viruses. Humans and other mammals are their natural hosts, and the viruses are spread widely via arthropods (e.g. ticks and mosquitoes). The Flaviviridae family is classified into four genera, including *Flavivirus*, *Pestivirus*, *Hepacivirus* and *Pegivirus*, and over 100 species. The *Flavivirus* genus includes the well-understood yellow fever virus, Japanese encephalitis virus (JEV), dengue virus, West Nile virus and Zika virus. The *Pestivirus* genus includes bovine viral diarrhea virus. The *Hepacivirus* genus is classified into 14 species which are abbreviated as the hepatitis viruses A, B, C... through N, wherein the well-known one is the hepatitis C virus. The *Pegivirus* genus is classified into 11 species which are abbreviated as the hepatotropic viruses A, B, C... through K.

Dengue fever, which mainly occurs in the subtropical and tropical countries, is an acute infectious disease caused by the dengue virus, and spread to humans via mosquitoes, e.g. *Aedes aegypti* and *Aedes albopictus.* The dengue viruses are classified into four types, i.e. I, II, III and IV, according to different virus serotypes, and each serotype has the abilities of infection and pathogenesis.

The latency period for the dengue fever is typically about 3 days to 8 days (the longest one being up to 14 days). The period between the day before illness and five days after illness is called "the communicability period" or "viremia". Dengue viruses exist in the patient's blood. Mosquitoes, *A. aegypti* or *A*. *albopictus*, will take up the dengue viruses if the patients are bitten by the mosquitoes during the communicability period. The dengue viruses enrich inside the mosquitoes during an 8-to-12-day multiplication, and the mosquitoes bite other people causing the dengue viruses to spread.

Some patients have mild syndromes during the dengue fever infection, and some do not even appear to show any symptoms. The typical symptoms of dengue fever include the phenomena such as a sudden fever higher than 38°C, headache, posterior eye pain, muscle soreness, arthralgia, rash, drowsiness, restlessness, liver enlargement, the increased vascular permeability, plasma leakage, bleeding, severe lethal bleeding and so on. Furthermore, if patients are infected with different dengue virus serotypes (DENV) for a consecutive two times, in particular the patients who are infected with the DENV-1 firstly followed by the DENV-2 or DENV-3 infection, or the patients who are infected with the DENV-3 firstly followed by the DENV-2 infection, the possibility of causing the dengue hemorrhagic fever (DHF) and dengue shock syndrome (DSS) is significantly increased to result in more severe clinical syndromes, such as shock or severe complications. Mortality is up to more than 20% if timely treatment is absent.

There is no sovereign remedy for treating dengue fever. In the prevention of dengue fever, Sanofi Pasteur (Lyon, France) developed the first dengue fever vaccine (a tetravalent attenuated live vaccine) in the world for people between the ages of 9 to 45 years old. However, this vaccine generated poor protection against DENV-2. In addition, GlaxoSmithKline (GSK, Brentford, the United Kingdom) also developed a deactivated vaccine, TDEN-PIV, that progressed to phase II clinical trials.

The World Health Organization (WHO) classified dengue fever patients into groups A, B and C depending on the occurrence of "warning sign" or not (Dengue Guidelines for Diagnosis, Treatment, Prevention and Control, 2009, World Health Organization, ISBN 978 92 4 154787 1), rather than the simple and traditional classification for dengue fever patients and DHF patients. The warning sign means that a dengue fever patient shows abdominal pain or abdominal tenderness, persistent vomiting, clinical fluid accumulation (e.g. ascites, pleural effusion and so on), mucosal bleed, lethargy, restlessness, the liver enlargement more than 2 cm, an increase in the hematocrit (HCT) concurrent with a rapid decrease in the platelet count, while keeping in mind co-existing conditions (e.g. diabetes, renal failure, chronic hemolytic disease, obesity, pregnancy, infancy, and old age), and adverse social circumstances (such as living alone, or living far from hospital).

Among these, patients in group A have neither "warning signs", co-existing conditions nor adverse social circumstances; patients in group B have "warning signs", and have the features of co-existing conditions and/or adverse social circumstances; and patients in group C have severe plasma leakage with shock and/or fluid accumulation with respiratory distress, severe bleeding, and/or organ impairment (such as liver function impairment, central nervous system impairment, heart failure, renal failure, cardiomyopathy, encephalopathy, encephalitis and so on).

In the treatment, the patients in group A are monitored at home for disease progression; the patients in group B accept hospitalization; and the patients in group C need an emergency remedy or are transferred to the hospital with good equipment and experienced physicians and nurses.

The classification of dengue cases by the WTO (2009 Edition) was established to provide appropriate triage to assist the clinical remedy, so as to study the mechanism of the disease and evaluate medical intervention (such as intravenous dehydration, new anti-virus drugs and vaccines and so on) in the future.
Honasawek et al describes that serum Hyaluronan (SA) levels can be used to diagnose dengue fever patients. SA levels are measured using biotinylated HA.

It is therefore the Applicant's attempt to deal with the above situation encountered in the prior art.
The present invention relates to a pharmaceutical composition for blocking a hyaluronan in a serum of a subject to prevent an inflammation thereof or treating the subject suffering from an infection of a dengue virus, wherein the subject was identified to suffer from a dengue virus infection, the pharmaceutical composition characterized by comprising:
a component being one selected from the group consisting of a first pharmaceutically effective amount of a 4-methyl umbelliferone sodium salt, a second pharmaceutically effective amount of a CD44 small interfering RNA (siRNA), a third pharmaceutically effective amount of an anti-CD44 antibody, a fourth pharmaceutically effective amount of a hyaluronidase and a combination thereof.

To assist doctors to evaluate the subsequent illness course of patients with dengue fever at his/her early stage of illness, and to adopt the appropriate treatment or therapy, novel and progressive techniques are developed in the present invention.

Described and not claimed *per se* is a method to determine whether a warning sign will occur in a subject with a *Flavivirus* infectious illness, and the method includes steps of: (a) providing a serum of the subject; (b) measuring a level of a hyaluronan in the serum; and (c) determining that the warning sign will occur in an illness course of the subject when the level is higher than or equal to 70 ng/mL.

In one embodiment, the subject is a human, and a period that the human is infected with dengue fever is at an early stage of the illness of the human.

In one embodiment, the human with the dengue fever shows the warning sign in the illness course, and the warning sign includes an abdominal pain, an abdominal tenderness, a persistent vomiting, a clinical fluid accumulation, a mucosal bleed, a lethargy, a restlessness, a liver enlargement of more than 2 centimeters, an increase in a hematocrit (HCT) concurrent with a rapid decrease in a platelet count, and a combination thereof.

Described and not claimed *per se* is that the step (b) is performed in a well of a microplate where an aggrecan is coated thereon already, and the step (b) further includes: (b1) adding the serum to cause the hyaluronan which may exist in the serum to be bound to the aggrecan; (b2) adding a biotinylated-proteoglycan to be bound to the hyaluronan; (b3) adding a streptavidin-conjugated enzyme to be bound to the biotinylated-proteoglycan; (b4) adding a substrate to react with the streptavidin-conjugated enzyme to generate a chemiluminescent reaction; and (b5) determining an optical density at 450 nm to calculate the level of hyaluronan.

The present invention discloses a pharmaceutical composition for blocking a hyaluronan in a serum of a subject to prevent an inflammation thereof or treating the subject suffering from the *Flavivirus* infection, wherein the subject was identified to suffer from the *Flavivirus* infection. The pharmaceutical composition includes: a component being one selected from the group consisting of a first pharmaceutically effective amount of a 4-methyl umbelliferone sodium salt, a second pharmaceutically effective amount of a CD44 small interfering RNA (siRNA), a third pharmaceutically effective amount of an anti-CD44 antibody, a fourth pharmaceutically effective amount of a hyaluronidase and a combination thereof.

In one embodiment, the hyaluronan has a level in the serum of the subject higher than or equal to 70 ng/mL to indicate that a warning sign will occur in an illness course of the subject. In one embodiment, the pharmaceutical composition further includes a pharmaceutically acceptable carrier, an excipient, a diluents or an adjuvant. In one embodiment, the subject is a human.

In one embodiment, the first pharmaceutically effective amount is a first effective blood concentration of the 4-methyl umbelliferone sodium salt of the human being in a first range from 0.05 mM to 5 mM, the second pharmaceutically effective amount is a second effective blood concentration of the CD44 siRNA of the human being in a second range from 1 nM to 100 nM, the third pharmaceutically effective amount is a third effective blood concentration of the anti-CD44 antibody of the human being in a third range from 5 µg/mL to 500 µg/mL, and the fourth pharmaceutically effective amount is a fourth effective blood concentration of the hyaluronidase of the human being in a fourth range from 0.5 unit/mL to 50 units/mL.

The objectives and advantages of the present invention will become more readily apparent to those ordinarily skilled in the art after reviewing the following detailed descriptions and accompanying drawings.
Fig. 1 illustrates a diagram showing a receiver operating characteristic (ROC) curve for the level of serum hyaluronan (SA) of the dengue fever patients at the early stage of the illness in the present invention.
Fig. 2 illustrates a diagram showing the ratio of the warning signs (WS)-positive to the WS-negative in the dengue fever patients during their whole illness courses in the present invention.
Fig. 3 illustrates a diagram showing the platelet (PLT) nadir (smaller than or not smaller than 50,000/µl) of the dengue fever patients during their whole illness courses in the present invention.
Fig. 4 illustrates a diagram showing the blood concentration phenomenon for the increased hematocrit (HCT) level (>20%) appearing in the dengue fever patients during their whole illness courses in the present invention.
Fig. 5 illustrates a diagram showing the Akt phosphorylation in the human vascular endothelial cells being subjected to the dengue virus (DENV) nonstructural protein 1 (NS1) or the pharmaceutical composition of the present invention.
Fig. 6 illustrates a diagram showing the transwell permeability assay of human microvascular endothelial cells.

The present invention will now be described more specifically with reference to the following embodiments. It is to be noted that the following descriptions of the preferred embodiments of this invention are presented herein for purpose of illustration and description only; they are not intended to be exhaustive or to be limited to the precise form disclosed.

### Definition:

The illnesses of the dengue fever patients herein are classified into "dengue fever", "severe dengue", "dengue hemorrhagic fever (DHF)", "dengue shock syndrome (DSS)" and so on according to the clinical manifestations or examination results.

The definition of the term "severe dengue" herein conforms to one or more items as follows: severe plasma leakage with shock, severe plasma leakage with fluid accumulation and respiratory distress, severe bleeding evaluation, severe organ impairment, glutamic oxaloacetic transaminase (GOT) or glutamic pyruvic transaminase (GPT) ≥ 1,000 IU/L in the liver, consciousness impairment in the central nervous system, heart failure or others.

According to the classification of the dengue cases by the WHO (2009 Edition), the term "warning sign" herein is defined as that dengue fever patients show abdominal pain or abdominal tenderness, persistent vomiting, clinical fluid accumulation, mucosal bleeding, lethargy, restlessness, liver enlargement of more than 2 cm, and an increase in the hematocrit (HCT) concurrent with a rapid decrease in the platelet count.

In the embodiment of the present invention, the patients who are infected with dengue virus and identified to have dengue fever are the population, and the levels of hyaluronan in the sera of the patients are measured and statistically calculated to define that the warning sign will occur in the illness course of the patients when the level is higher than or equal to 70 ng/mL. Because the diseases caused by the yellow fever virus, JEV, West Nile virus and Zika virus (where all belong to the *Flavivirus*) also make patients have symptoms similar to dengue fever, the technique in the present invention can also be used in patients who suffer from yellow fever, Japanese encephalitis, West Nile fever and Zika virus infection.

### Embodiment 1:

### Experimental methods:

To determine whether a warning sign will occur in a patient with a *Flavivirus* infectious illness, the level of SA in the patient was determined using the Hyaluronan DuoSet^{®} ELISA development system (Cat. No. DY3614-05, R&D systems, Inc., U.S.A.). The skilled person in the art can implement Embodiment 1 in view of the manufacturer's instructions of the Hyaluronan DuoSet^{®} ELISA development system, or perform the experiments by preparing the materials and reagents by himself/herself and using the same experimental methods.

First, a 96-well microplate for the enzyme-linked immunosorbent assay (ELISA) was prepared as follows. The hyaluronan capture reagent (i.e. recombinant human aggrecan) was diluted to a working concentration in the phosphate-buffered saline (PBS, 137 mM NaCl, 2.7 mM KCl, 8.1 mM Na₂HPO₄, 1.5 mM KH₂PO₄, pH 7.2-7.4, 0.2 µm filtered) without carrier protein. The 96-well microplate with 100 µL per well of the diluted hyaluronan capture reagent was immediately coated. The microplate was sealed and incubated overnight at room temperature.

Each well was aspirated and washed with a wash buffer (0.05% Tween^{®} 20 in PBS, pH 7.2-7.4), the process was repeated two times for a total of three washes. In detail, each well was washed by filling with the wash buffer (400 µL) using a squirt bottle, a manifold dispenser or an autowasher. Complete removal of liquid at each step was essential for good performance. After the last wash, any remaining wash buffer was removed by aspirating or by inverting the microplate and blotting it against clean paper towels. The microplate was blocked by adding 300 µL of a reagent diluent (5% Tween^{®} 20 in PBS, pH 7.2-7.4, 0.2 µm filtered) to each well. The microplate was incubated at room temperature for a minimum of 1 hour. The aspiration/wash was repeated. The microplate was now ready for the sample addition and the ELISA experiment.

A 100-µL sample or hyaluronan standards in the reagent diluent (or an appropriate diluent) per well was added to the well. An adhesive strip was covered on the microplate, and the microplate was incubated for 2 hours at room temperature. The aspiration/wash was repeated. A 100-µL biotinylated detection reagent (i.e. the biotinylated recombinant human proteoglycan) which was diluted in the reagent diluent was added to each well. A new adhesive strip was covered on the microplate, which was further incubated for 2 hours at room temperature. A 100-µL working dilution of streptavidin-horseradish peroxidase (HRP) was added to each well. The microplate was covered and incubated for 20 minutes at room temperature. Placing the microplate in direct light was avoided. The aspiration/wash was repeated. A 100-µL substrate solution (1:1 (v/v) mixture of color reagent A (H₂O₂) and color reagent B (tetramethylbenzidine)) was added to each well, and the microplate was incubated for 20 minutes at room temperature. Placing the microplate in direct light was avoided. A 50-µL stop solution (2 N H₂SO₄) was added to each well. The microplate was gently tapped to ensure thorough mixing. The optical density of each well was determined immediately using a microplate reader set to 450 nm.

A six point standard curve of the hyaluronan standard using 3-fold serial dilutions in the reagent diluent was recommended. Thus, the concentration of the hyaluronan standard was 90, 30, 10, 3.33, 1.11 and 0.370 ng/mL. A standard curve was plotted based on the concentration of the hyaluronan standard and its average optical density.

### Experimental results:

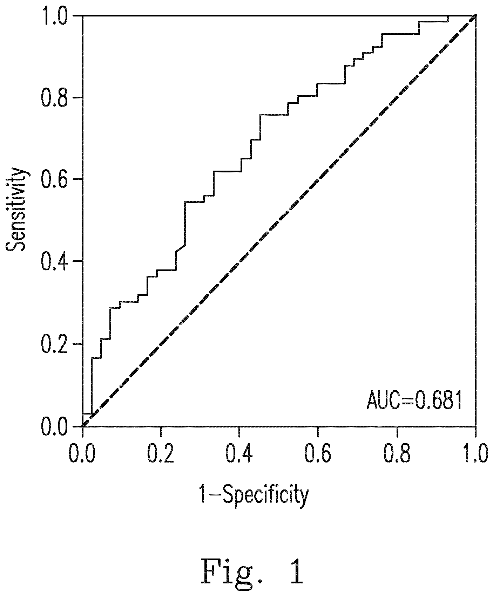

Please refer to Fig. 1, which illustrates a diagram showing a receiver operating characteristic (ROC) curve for the level of SA in the dengue fever patients at the early stage of the illness in the present invention. When the serum sample of each dengue fever patient (n = 108) was measured, a critical value whether the warning sign will occur was precisely determined by the ROC curve depending on whether the patients show the warning sign. As shown in Fig. 1, an area under curve (AUC) is 0.681 (95% confidence interval (C.I.) = 0.58-0.78, p value = 0.002), the best critical value is 70.06 ng/mL, the sensitivity is 0.758, and the 1-specificity is 0.548. Therefore, in the subsequent analysis, the level of SA which is higher than or equal to 70 ng/mL is the critical value for determining whether the warning sign will occur in the whole illness course of the patient.

Please refer to Table 1, which is the univariate analysis of factors associated with the dengue fever patients presented with warning signs (WS) throughout the illnesses (n = 108). The levels of hyaluronan in the sera of the dengue fever patients (n = 108) were measured during the febrile phase (at early illness stage), and their illness progression was traced until recovery. If the level of SA in the dengue fever patient is higher than or equal to 70 ng/mL during the febrile phase, the possibility that they show "the warning sign" in whole illness course is 3.78 times higher than the patients with the level lower than 70 ng/mL (p = 0.003, 95% C.I. = 1.65-8.66). The results of the univariate analysis in Table 1 also showed that the age ≥ 65 years, the secondary dengue viral infection, and the level of SA ≥ 70 ng/mL (at the febrile phase) are prediction factors for predicting whether the dengue fever patients belong to the "WS-positive" group. Furthermore, important prediction factors (i.e. patient's age, and secondary infection) were calibrated using multivariate analysis, and the results still showed that the level of SA ≥ 70 ng/mL in the dengue fever patient at the early stage (the febrile phase) is an independent prediction factor for predicting whether the warning sign will occur in the illness course of the patient (referring to Table 2). Therefore, depending on the level of SA (≥ or < 70 ng/mL) in the dengue fever patient at the early illness stage, doctors can determine whether the warning sign will occur in his/her illness course, and further evaluate the patient's subsequent conditions so as to provide him/her with the appropriate remedy or treatment.

**Table 1. Univariate analysis of factors associated with dengue fever patients presented with warning signs (WS) throughout the illnesses (n = 108)**

| Variables | | No. (%) of patients | | Odds ratio (95% Confidence interval) | *p-*value |
|---|---|---|---|---|---|
| | | WS-positive (n = 66) | WS-negative (n = 42) | | |
| **Demographic characteristics** | | | | | |
| | Gender, female | 35 (53.0) | 19 (45.2) | 1.37 (0.63-2.97) | 0.554 |
| | Age ≥ 65 years | 34 (51.5) | 10 (23.8) | 3.40 (1.44-8.02) | 0.008 |
| | Secondary infection | 43 (65.2) | 17 (40.5) | 2.75 (1.24-6.10) | 0.020 |

| **Comorbidities** | | | | | |
|---|---|---|---|---|---|
| | Diabetes | 16 (24.2) | 7 (16.7) | 1.60 (0.60-4.30) | 0.486 |

| **Laboratory data** | | | | | |
|---|---|---|---|---|---|
| | Thrombocytopenia (first sampling) | 26 (39.4) | 11 (26.2) | 1.83 (0.79-4.27) | 0.230 |
| | SA (febrile phase) ≥ 70 ng/ml | 50 (75.8) | 19 (45.2) | 3.78 (1.65-8.66) | 0.003 |

**Table 2. Multivariate analysis of the serum samples of the dengue fever patients (n = 108)**

| Variable | Adjusted odds ratio (95 % CI) | *p*-value |
|---|---|---|
| Age ≥ 65 years | 2.01 (0.78-5.21) | 0.151 |
| Secondary infection | 2.00 (0.84-4.76) | 0.118 |
| Serum HA (febrile phase) ≥70 ng/ml | 2.80 (1.15-6.80) | 0.023 |

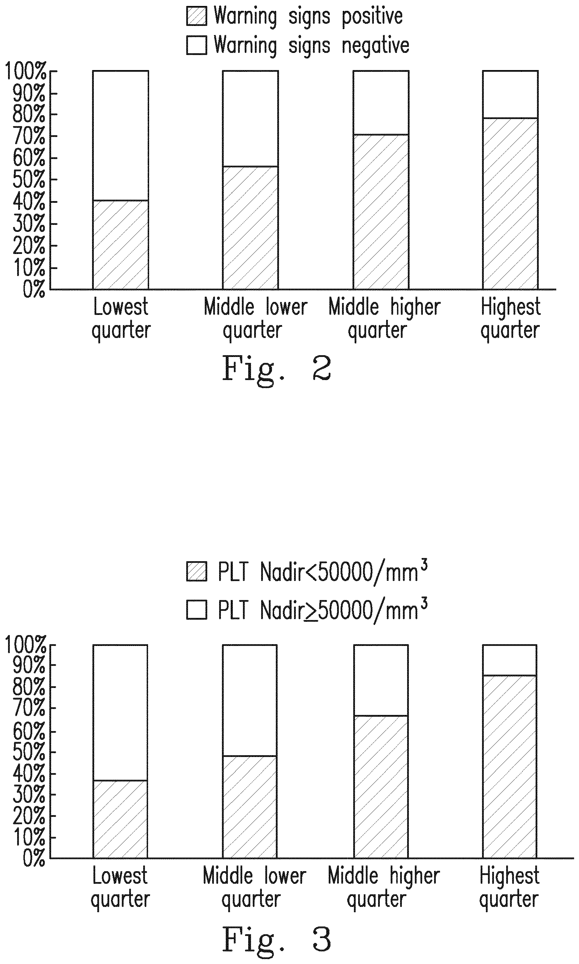

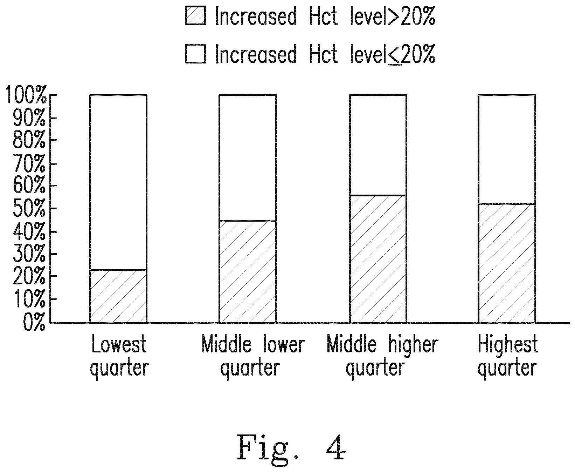

Please refer to Fig. 2 to Fig. 4, which illustrates diagrams showing (a) the ratio of the warning signs (WS)-positive to the WS-negative, (b) the platelet (PLT) nadir (< or ≥ 50,000/µl), and (c) the blood concentration phenomenon for the increased hematocrit (HCT) level (>20%) in the dengue fever patients during their whole illness course in the present invention. These 108 dengue fever patients were classified into four groups (i.e. lowest quarter, middle lower quarter, middle higher quarter, and highest quarter) depending on the levels of SA at their early illness stage (the febrile stage). It can be known from Figs. 2 to 4 that, compared to the group with the lower level of SA, the group with the higher level of SA has a high possibility of having the warning sign in the whole illness course, and the PLT nadir < 50,000/µl and the increased hematocrit (HCT) level (>20%).

### Embodiment 2:

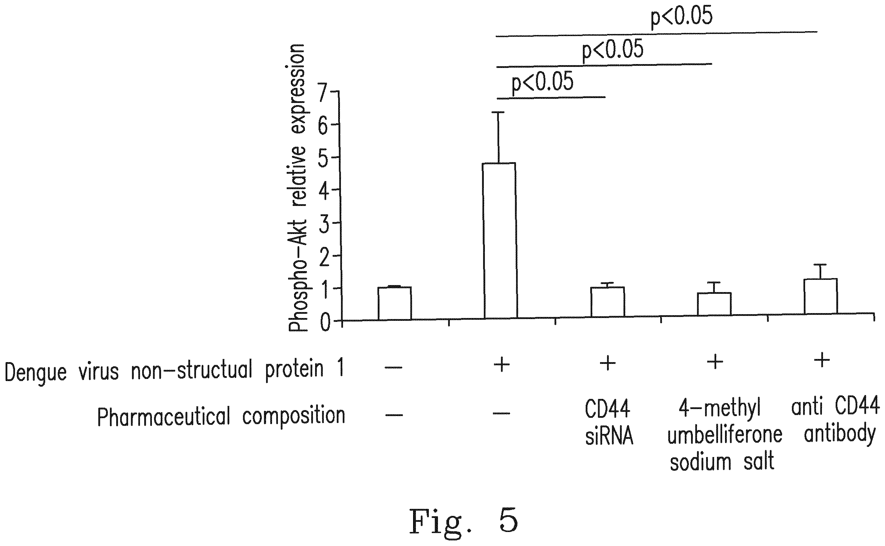

Based on whether the warning sign will occur in the illness course of the patent with the level of SA ≥ 70 ng/mL, a pharmaceutical composition for blocking the SA in the patient to prevent inflammation is disclosed in this Embodiment. The pharmaceutical composition includes a pharmaceutically effective amount of 4-methyl umbelliferone sodium salt, wherein the therapeutically effective amount is an effective blood concentration of 4-methyl umbelliferone sodium salt of the patient being in a range from 0.05 mM to 5 mM. The pharmaceutical composition may further include a pharmaceutically acceptable carrier, an excipient, a diluent, or an adjuvant. Please refer to Fig. 5, which illustrates a diagram showing the Akt phosphorylation in the human vascular endothelial cells being subjected to the dengue virus (DENV) nonstructural protein 1 (NS1) or the pharmaceutical composition of the present invention. In Fig. 5, the human vascular endothelial cells (4 × 10⁵ cells cultivated in a 60-mm dish) were treated with a nonstructural protein 1 (3 µg/ml, Cat. No. ENZ-PRT105-0100, Enzo Life Sciences, Inc., NY, U.S.A.), and the Akt phosphorylation was significantly induced in the cells, indicating that the dengue virus results in inflammation in the human vascular endothelial cells. In addition, the human vascular endothelial cells were treated with 4-methyl umbelliferone sodium salt for 12 hours followed by the DENV NS1 treatment. Subsequently, the cellular proteins were extracted and subjected to the sodium dodecyl sulfate polyacrylamide gel electrophoresis (SDS-PAGE) to separate the proteins according to their molecular weights. Next, the proteins in the gel were transferred to a polyvinylidene difluoride (PVDF) membrane, and the phospho-Akt (p-Akt) signal on the PVDF membrane was detected using the Western blotting and the p-Akt antibody. The results showed that a range from 0.05 mM to 5 mM is the effective blood concentration of 4-methyl umbelliferone sodium salt in the dengue fever patient, indicating that 4-methyl umbelliferone sodium salt can be used to inhibit the inflammation of the human vascular endothelial cells caused by the dengue virus (referring to Fig. 5).

### Embodiment 3:

The CD44 antigen, plays important roles in many biological functions (such as inflammation), is a surface glycoprotein on mammalian cells, and hyaluronan is a main molecule to bound with the CD44 antigen. Thus, an abundance of hyaluronan would bind to CD44 to influence CD44's biological functions when the level of SA in the dengue fever patient is too high. In this Embodiment, CD44 small interfering RNA (siRNA) was used to inhibit the CD44 expression of the vascular endothelial cells of the dengue fever patients, and block the combination between hyaluronan and CD44. In this Embodiment, the experimental method for inhibiting cellular protein expression using siRNA was known to the skilled person in the art, and the CD44 siRNAs were the artificially synthesized SEQ ID NO:1 (5'-GAAUAUAACC UGCCGCUUU-3'), SEQ ID NO: 2 (5'-CAAGUGGACU CAACGGAGA-3'), SEQ ID NO: 3 (5'-CGAAGAAGGU GUGGGCAGA-3') and SEQ ID NO: 4 (5'-GAUCAACAGU GGCAAUGGA-3'). Furthermore, two, three or four siRNAs in the SEQ ID NOs: 1 to 4 may be combined to inhibit the CD44 expression of the vascular endothelial cells (4 × 10⁵ cells cultivated in a 60-mm dish) of the dengue fever patient. First, in view of the manufacturer's instructions of siLentFect^{™} Lipid Reagent (Bio-Rad laboratories AB, Sweden), the CD44 siRNA was mixed with siLentFect^{™} Lipid Reagent, the mixture was transfected into the human vascular endothelial cells for 24 hours followed by the DENV NS1 treatment (3 µg/ml, Cat. No. ENZ-PRT105-0100, Enzo Life Sciences, Inc., NY, U.S.A.). Subsequently, the cellular proteins were extracted and subjected to the SDS-PAGE to separate the proteins according to their molecular weights. Next, the proteins in the gel were transferred to a PVDF membrane, and the p-Akt signal on the PVDF membrane was detected using the Western blotting and the p-Akt antibody. The results showed that a range from 1 nM to 100 nM is an effective blood concentration of CD44 siRNA (SEQ ID NOs: 1 to 4) in the dengue fever patient, indicating that the CD44 siRNA can be used to inhibit the inflammation of the vascular endothelial cells in humans caused by the dengue virus (referring to Fig. 5). Therefore, the CD44 siRNA (SEQ ID NOs: 1 to 4) of the present invention can be used to prepare a pharmaceutical composition for treating inflammation in dengue fever patients.

### Embodiment 4:

In this Embodiment, an anti-CD44 antibody (MA4400, Invitrogen, CA, U.S.A.) of 5 µg/ml to 500 µg/ml was used to inhibit the CD44 surface antigen of the vascular endothelial cells of the dengue fever patients. First, the human vascular endothelial cells (4 × 10⁵ cells cultivated in a 60-mm dish) were treated with the anti-CD44 antibody for 12 hours followed by the treatment of DENV NS1 (3 µg/ml, Cat. No. ENZ-PRT105-0100, Enzo Life Sciences, Inc., NY, U.S.A.). Subsequently, the cellular proteins were extracted and subjected to the SDS-PAGE to separate the proteins according to their molecular weights. Next, the proteins in the gel were transferred to a PVDF membrane, and the p-Akt signal on the PVDF membrane was detected using Western blotting and the p-Akt antibody. The results showed that a range from 5 µg/ml to 500 µg/ml is an effective blood concentration of anti-CD44 antibody in the dengue fever patient to inhibit p-Akt in the vascular endothelial cells of the dengue fever patients, indicating that the anti-CD44 antibody can be used to inhibit the inflammation of the vascular endothelial cells in humans caused by the dengue virus (referring to Fig. 5). Therefore, the anti-CD44 antibody of the present invention can be used to prepare a pharmaceutical composition for treating inflammation in dengue fever patients.

### Embodiment 5:

Vascular leakage is an important feature in several diseases, such as septic shock, viral hemorrhagic fever, cancer metastasis and ischemia-reperfusion injuries. Thus, an *in vitro* endothelial permeability assay will provide insight into the establishment or progression of such diseases. The transwell permeability assays directly detect the penetration of a specific molecule, which can be detected by a spectrometer-based absorbance reader.

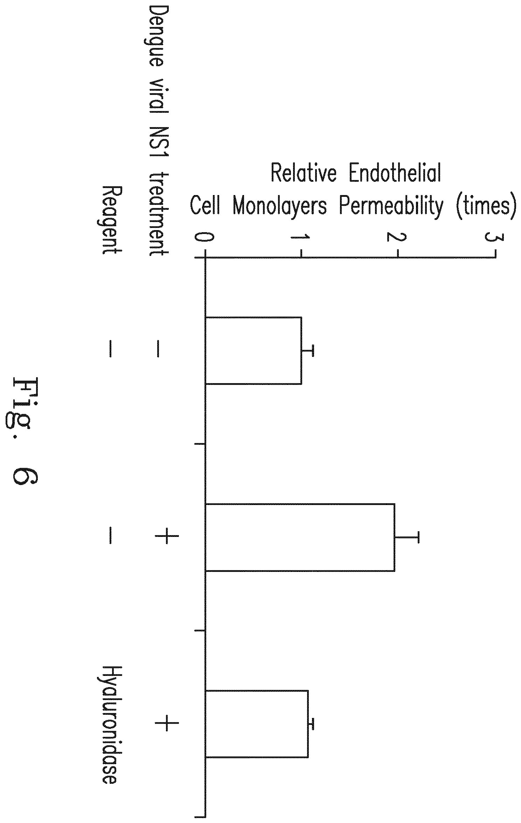

The permeability of human microvascular endothelial cells treated or not with DENV NS1 (3 µg/ml, Cat. No. ENZ-PRT105-0100, Enzo Life Sciences, Inc., NY, U.S.A.), was measured by an *in vitro* transwell permeability assay that mimics human endothelium *in vivo* (referring to http://www.bio-protocol.org/e2273). In brief, human microvascular endothelial cells (2 × 10⁵) were grown in a 24-Transwell^{®} (pore size: 0.4 µm, Cat. No. 3414; Corning, Kennebunk, ME, U.S.A.) for 5 days until a confluent monolayer was formed. Next, some of the samples were treated for 6 hours with DENV NS1 protein in the absence or presence of exogenously added hyaluronidase (0.5 unit/mL to 50 units/mL), media was aspirated, and 200 µL fresh serum-free medium containing 3 µL of streptavidin-horseradish peroxidase (HRP) (GE Healthcare, Cat. No. RPN1231, UK) was added. To measure the HRP that had leaked through the endothelial layer, the inserts were moved to a new 24-well plate with 500 µl of serum-free medium, covered and placed in a 37°C incubator for 5 minutes. Twenty microliters of medium, from each lower chamber, was transferred to a 96-well plate and analyzed for HRP activity by adding 100 µL 3,3',5,5'-tetramethylbenzidine (TMB) substrate (Sigma-Aldrich, Cat. No. T4444, St. Louis, MO, U.S.A.). Color development was detected by an EnSpire Multimode Reader (PerkinElmer, Upplands Väsby, Sweden) at 450 nm. The results showed that the concomitantly added hyaluronidase could reverse the DENV NS1-induced endothelial hyper-permeability significantly (referring to Fig. 6).

Embodiments 6 to 11 are not encompassed by the wording of the claims, but are considered useful for understanding of the invention

6. Described and not claimed *per se* is a method to determine whether a warning sign will occur in a subject with a *Flavivirus* infectious illness, the method characterized by comprising steps of:
(a) providing a serum of the subject;
(b) measuring a level of a hyaluronan in the serum; and
(c) determining that the warning sign will occur in an illness course of the subject when the level is higher than or equal to 70 ng/mL.

7. Described and not claimed *per se* is the method according to 6, characterized in that the subject is a human, and a period that the human is infected with a *Flavivirus* virus is at an early stage of the illness of the human.

8. Described and not claimed *per se* is the method according to 7, characterized in that the *Flavivirus* virus is a species being one selected from the group consisting of a yellow fever virus, a Japanese encephalitis virus (JEV), a dengue virus, a West Nile virus and a Zika virus.

9. Described and not claimed *per se* is the method according to 8, characterized in that the yellow fever virus causes the human to suffer from yellow fever, the JEV causes the human to suffer from Japanese encephalitis, the dengue virus causes the human to suffer from dengue fever, the West Nile virus causes the human to suffer from West Nile fever, and the Zika virus causes the human to suffer from a Zika virus infection.

10. Described and not claimed *per se* is the method according to 9, characterized in that the human with the dengue fever shows the warning sign in the illness course, and the warning sign is one selected from the group consisting of an abdominal pain, an abdominal tenderness, a persistent vomiting, a clinical fluid accumulation, a mucosal bleed, a lethargy, a restlessness, a liver enlargement of more than 2 centimeters, an increase in a hematocrit (HCT) concurrent with a rapid decrease in a platelet count, and a combination thereof.

11. Described and not claimed *per se* is the method according to 6, characterized in that the step (b) is performed in a well of a microplate where an aggrecan is coated thereon already, and the step (b) further comprises:
(b1) adding the serum to cause the hyaluronan which may exist in the serum to be bound to the aggrecan;
(b2) adding a biotinylated-proteoglycan to be bound to the hyaluronan;
(b3) adding a streptavidin-conjugated enzyme to be bound to the biotinylated-proteoglycan;
(b4) adding a substrate to react with the streptavidin-conjugated enzyme to generate a chemiluminescent reaction; and
(b5) determining an optical density at 450 nm to calculate the level of hyaluronan.

The present invention discloses:
12. A pharmaceutical composition for blocking a hyaluronan in a serum of a subject to prevent an inflammation thereof or treating the subject suffering from an infection of a dengue virus, wherein the subject was identified to suffer from a dengue virus infection, the pharmaceutical composition characterized by comprising:
a component being one selected from the group consisting of a first pharmaceutically effective amount of a 4-methyl umbelliferone sodium salt, a second pharmaceutically effective amount of a CD44 small interfering RNA (siRNA), a third pharmaceutically effective amount of an anti-CD44 antibody, a fourth pharmaceutically effective amount of a hyaluronidase and a combination thereof.

13. The pharmaceutical composition according to Embodiment 12, characterized in that the hyaluronan has a level in the serum of the subject higher than or equal to 70 ng/mL to indicate that a warning sign will occur in an illness course of the subject.

14. The pharmaceutical composition according to Embodiment 12, characterized in that the pharmaceutical composition further comprises one selected from the group consisting of a pharmaceutically acceptable carrier, an excipient, a diluent, an adjuvant and a combination thereof.

15. The pharmaceutical composition according to Embodiment 12, characterized in that the subject is a human.

16. The pharmaceutical composition according to Embodiment 15, characterized in that the first pharmaceutically effective amount is a first effective blood concentration of the 4-methyl umbelliferone sodium salt of the human being in a first range from 0.05 mM to 5 mM, the second pharmaceutically effective amount is a second effective blood concentration of the CD44 siRNA of the human being in a second range from 1 nM to 100 nM, the third pharmaceutically effective amount is a third effective blood concentration of the anti-CD44 antibody of the human being in a third range from 5 µg/mL to 500 µg/mL, and the fourth pharmaceutically effective amount is a fourth effective blood concentration of the hyaluronidase of the human being in a fourth range from 0.5 unit/mL to 50 units/mL.

## Claims

1. A pharmaceutical composition for use in blocking a hyaluronan in a serum of a subject to prevent an inflammation thereof or treating the subject suffering from an infection with a dengue virus, wherein the subject was identified to suffer from a dengue virus infection, the pharmaceutical composition **characterized by** comprising:
a component being one selected from the group consisting of a first pharmaceutically effective amount of a 4-methyl umbelliferone sodium salt, a second pharmaceutically effective amount of a CD44 small interfering RNA (siRNA), a third pharmaceutically effective amount of an anti-CD44 antibody, a fourth pharmaceutically effective amount of a hyaluronidase and a combination thereof.

2. The pharmaceutical composition for use according to Claim 1, **characterized in that** the hyaluronan has a level in the serum of the subject higher than or equal to 70 ng/mL to indicate that a warning sign will occur in an illness course of the subject.

3. The pharmaceutical composition for use according to Claim 1, **characterized in that** the pharmaceutical composition further comprises one selected from the group consisting of a pharmaceutically acceptable carrier, an excipient, a diluent, an adjuvant and a combination thereof.

4. The pharmaceutical composition for use according to Claim 1, **characterized in that** the subject is a human.

5. The pharmaceutical composition for use according to Claim 4, **characterized in that** the first pharmaceutically effective amount is a first effective blood concentration of the 4-methyl umbelliferone sodium salt of the human being in a first range from 0.05 mM to 5 mM.

6. The pharmaceutical composition for use according to Claim 4, **characterized in that** the second pharmaceutically effective amount is a second effective blood concentration of the CD44 siRNA of the human being in a second range from 1 nM to 100 nM.

7. The pharmaceutical composition for use according to Claim 4, **characterized in that** the third pharmaceutically effective amount is a third effective blood concentration of the anti-CD44 antibody of the human being in a third range from 5 µg/mL to 500 µg/mL.

8. The pharmaceutical composition for use according to Claim 4, **characterized in that** the fourth pharmaceutically effective amount is a fourth effective blood concentration of the hyaluronidase of the human being in a fourth range from 0.5 unit/mL to 50 units/mL.

9. The pharmaceutical composition for use according to Claim 4, **characterized in that** the human with the dengue fever shows the warning sign in the illness course, and the warning sign is one selected from the group consisting of an abdominal pain, an abdominal tenderness, a persistent vomiting, a clinical fluid accumulation, a mucosal bleeding, a lethargy, a restlessness, a liver enlargement of more than 2 centimeters, an increase in a hematocrit (HCT) concurrent with a rapid decrease in a platelet count, and a combination thereof.

10. The pharmaceutical composition for use according to Claim 1, **characterized in that** the level of the hyaluronan in the subject is determined using the enzyme-linked immunosorbent assay (ELISA).

11. The pharmaceutical composition for use according to Claim 1, wherein the CD44 siRNA is one selected from the group consisting of an SEQ ID NO:1, an SEQ ID NO: 2, an SEQ ID NO: 3, an SEQ ID NO: 4 and the combination thereof.

## Patentansprüche

1. Pharmazeutische Zusammensetzung zur Verwendung bei der Blockierung eines Hyaluronans in einem Serum eines Individuums, um eine Entzündung davon zu verhindern, oder bei der Behandlung des Individuums, dass an einer Infektion mit einem Dengue-Virus leidet, wobei festgestellt wurde, dass das Individuum an einer Dengue-Virus-Infektion leidet, wobei die pharmazeutische Zusammensetzung **dadurch gekennzeichnet ist, dass** sie umfasst:
eine Komponente, die ausgewählt ist aus der Gruppe bestehend aus einer ersten pharmazeutisch wirksamen Menge eines Natriumsalzes von 4-Methylumbelliferon, einer zweiten pharmazeutisch wirksamen Menge einer kleinen interferierenden CD44-RNA (siRNA), einer dritten pharmazeutisch wirksamen Menge eines Anti-CD44-Antikörpers, einer vierten pharmazeutisch wirksamen Menge einer Hyaluronidase und einer Kombination davon.

2. Pharmazeutische Zusammensetzung zur Verwendung nach Anspruch 1, **dadurch gekennzeichnet, dass** das Hyaluronan einen Spiegel in dem Serum des Individuums, der höher als oder gleich 70 ng/ml ist, aufweist, um anzuzeigen, dass ein Warnzeichen in einem Krankheitsverlauf des Individuums auftreten wird.

3. Pharmazeutische Zusammensetzung zur Verwendung nach Anspruch 1, **dadurch gekennzeichnet, dass** die pharmazeutische Zusammensetzung weiterhin eines, ausgewählt aus der Gruppe bestehend aus einem pharmazeutisch akzeptablen Träger, einem Exzipienten, einem Verdünnungsmittel, einem Adjuvans und einer Kombination davon, umfasst.

4. Pharmazeutische Zusammensetzung zur Verwendung nach Anspruch 1, **dadurch gekennzeichnet, dass** das Individuum ein Mensch ist.

5. Pharmazeutische Zusammensetzung zur Verwendung nach Anspruch 4, **dadurch gekennzeichnet, dass** die erste pharmazeutisch wirksame Menge eine erste wirksame Konzentration des Natriumsalzes von 4-Methylumbelliferon im Blut des Menschen ist, die in einem ersten Bereich von 0,05 mM bis 5 mM liegt.

6. Pharmazeutische Zusammensetzung zur Verwendung nach Anspruch 4, **dadurch gekennzeichnet, dass** die zweite pharmazeutisch wirksame Menge eine zweite wirksame Konzentration der CD44-siRNA im Blut des Menschen ist, die in einem zweiten Bereich von 1 nM bis 100 nM liegt.

7. Pharmazeutische Zusammensetzung zur Verwendung nach Anspruch 4, **dadurch gekennzeichnet, dass** die dritte pharmazeutisch wirksame Menge eine dritte wirksame Konzentration des Anti-CD44-Antikörpers im Blut des Menschen ist, die in einem dritten Bereich von 5 µg/ml bis 500 µg/ml liegt.

8. Pharmazeutische Zusammensetzung zur Verwendung nach Anspruch 4, **dadurch gekennzeichnet, dass** die vierte pharmazeutisch wirksame Menge eine vierte wirksame Konzentration der Hyaluronidase im Blut des Menschen ist, die in einem vierten Bereich von 0,5 Einheiten/ml bis 50 Einheiten/ml liegt.

9. Pharmazeutische Zusammensetzung zur Verwendung nach Anspruch 4, **dadurch gekennzeichnet, dass** der Mensch mit Dengue-Fieber das Warnzeichen in dem Krankheitsverlauf zeigt, und das Warnzeichen eines, ausgewählt aus der Gruppe bestehend aus einem Bauchschmerz, einer Druckempfindlichkeit des Bauches, einem anhaltenden Erbrechen, einer klinischen Flüssigkeitsansammlung, einer Schleimhautblutung, einer Lethargie, einer Unruhe, einer Lebervergrößerung von mehr als 2 cm, einem Anstieg eines Hämatokriten (HCT) gleichzeitig mit einer rapiden Abnahme einer Thrombozytenzahl, und einer Kombination davon, ist.

10. Pharmazeutische Zusammensetzung zur Verwendung nach Anspruch 1, **dadurch gekennzeichnet, dass** der Hyaluronanspiegel bei dem Individuum unter Verwendung des Enzyme-Linked Immunosorbent Assay (ELISA) bestimmt wird.

11. Pharmazeutische Zusammensetzung zur Verwendung nach Anspruch 1, wobei die CD44-siRNA eine, ausgewählt aus der Gruppe bestehend aus einer SEQ ID Nr. 1, einer SEQ ID Nr. 2, einer SEQ ID Nr. 3, einer SEQ ID Nr. 4 und der Kombination davon, ist.

## Revendications

1. Composition pharmaceutique destinée à être utilisée dans le blocage d'un hyalurononane dans un sérum d'un sujet pour prévenir une inflammation de celui-ci ou traiter le sujet souffrant d'une infection à un virus de la dengue, le sujet ayant été identifié pour souffrir d'une infection par le virus de la dengue, la composition pharmaceutique **caractérisée** comme comprenant :
un composant qui est choisi dans le groupe constitué par une première quantité pharmaceutiquement efficace de sel de 4-méthyl umbelliférone de sodium, une deuxième quantité pharmaceutiquement efficace d'un petit ARN CD44 interférant (ARNsi), une troisième quantité pharmaceutiquement efficace d'un anticorps anti-CD44, une quatrième quantité pharmacetiquement efficace d'une hyaluronidase et une de leurs combinaisons.

2. Composition pharmaceutique destinée à être utilisée selon la revendication 1, **caractérisée en ce que** le hyaluronane a un niveau dans le sérum du sujet supérieur ou égal à 70 ng/mL pour indiquer qu'un signal d'avertissement se produira au cours d'une maladie du sujet.

3. Composition pharmaceutique destinée à être utilisée selon la revendication 1, **caractérisée en ce que** la composition pharmaceutique comprend en outre un choisi dans le groupe constitué par un véhicule, un excipient, un diluant, un adjuvant pharmaceutiquement acceptable et une de leurs combinaisons.

4. Composition pharmaceutique destinée à être utilisée selon la revendication 1, **caractérisée en ce que** le sujet est un être humain.

5. Composition pharmaceutique destinée à être utilisée selon la revendication 4, **caractérisée en ce que** la première quantité pharmaceutiquement efficace est une première concentration sanguine efficace du sel de 4-méhyl umbelliférone de l'être humain qui se situe dans une première plage de 0,05 mM à 5 mM.

6. Composition pharmaceutique destinée à être utilisée selon la revendication 4, **caractérisée en ce que** la deuxième quantité pharmaceutiquement efficace est une deuxième concentration sanguine efficace d'ARNsi CD44 de l'être humain qui se situe dans une deuxième plage de 1 nM à 100 nM.

7. Composition pharmaceutique destinée à être utilisée selon la revendication 4, **caractérisée en ce que** la troisième quantité pharmaceutiquement efficace est une troisième concentration sanguine efficace de l'aticorps anti-CD44 de l'être humain qui se situe dans une troisième plage de 5 µg/mL à 500 µg/mL.

8. Composition pharmaceutique destinée à être utilisée selon la revendication 4, **caractérisée en ce que** la quatrième quantité pharmaceutiquement efficace est une quatrième concentration sanguine efficace de la hyaluronidase de l'être humain qui se situe dans une quatrième plage de 0,5 unités/mL à 50 unités/mL.

9. Composition pharmaceutique destinée à être utilisée selon la revendication 4, **caractérisée en ce que** l'être humain avec la fièvre de dengue montre le signe d'avertissement dans le cours de la maladie, et le signal d'avertissement est un choisi dans le groupe constitué par une douleur abdominale, une tendresse abdominale, un vomissement persistent, une accumulation de fluide clinique, un saignement mucosal, une léthargie, une absence de repos, un élargissement du foie de plus de 2 centimètres, une augmentation dans un hématocrite (HCT) simultanément avec une diminution rapide d'un nombre de plaquettes et une de leurs combinaisons.

10. Composition pharmaceutique destinée à être utilisée selon la revendication 1, **caractérisée en ce que** le niveau du hyaluronane chez le sujet est déterminé en utilisant l'essai immunosorbant lié à une enzyme (enzyme-lnked immunosorbent assay, ELISA).

11. Composition pharmaceutique destinée à être utilisée selon la revendication 1, **caractérisée en ce que** l' ARNsi de CD44 est un choisi dans le groupe constitué par un de SEQ ID n° 1, SEQ ID n° 2, SEQ ID n° 3 et SEQ ID n° 4 et leur combinaison.
